# EUROPEAN PATENT APPLICATION

(11) **EP 3 311 842 A1**
(43) Date of publication of application: **25.04.2018**
(21) Application number: 17206128.5
(22) Date of filing: 13.06.2014
(51) Int. Cl.: A61K 45/06, A61K 31/42, A61K 31/498, A61P 3/10, A61P 9/10, A61P 5/50

(54) **COMPOSITIONS AND METHODS FOR TREATING METABOLIC DISORDERS**

(30) Priority: 13.06.2013 US 201361834864 P
(62) Divisional of application: 14811295.6
(71) Applicant: VeroScience LLC, Tiverton RI 02878 (US)
(72) Inventor: Cincotta, Anthony, H., Tiverton, RI Rhode Island (US); Zhang, Yahong, Westport, MA Massachusetts 02790 (US)
(74) Representative: Zacco Denmark A/S

(57) **Abstract**

The application discloses methods and compositions for regulating neuronal activities in the brain that are useful, for example, for treating metabolic disorders.

## Description

### FIELD OF THE INVENTION

This invention relates to methods and compositions for treating metabolic disorders.

### BACKGROUND

Metabolic disorder, such as, for example, diabetes and obesity, as well as metabolic syndrome, cardiovascular diseases, and pre-diabetes, affect millions of people, worldwide. Diabetes, one of the most insidious of the major diseases, can strike suddenly or lie undiagnosed for years while attacking the blood vessels and nerves. Diabetics, as a group, are far more often afflicted with blindness, heart disease, stroke, kidney disease, hearing loss, gangrene and impotence. One third of all visits to physicians in the U.S. are occasioned by diabetes and its complications. Diabetes and its complications are a leading cause of death in the U.S. and other countries.

Obesity, or excess fat deposits, is often associated with increasing cellular resistance to insulin, which precedes the onset of frank diabetes. Prior to the onset of diabetes, the pancreas of an obese individual is taxed to produce additional insulin; but eventually, perhaps over several years prior to the onset of frank type 2 diabetes, insulin productivity falls and diabetes results.

High fat diet-induced metabolic disorders are complex disorders associated with malfunction of multiple brain systems, especially altered circadian rhythmicity and dysfunction of arousal/reward regulation. Evidence exists indicating that that the circadian and arousal/reward systems interact with each other. The master circadian clock, the suprachiasmatic nucleus (SCN) of the hypothalamus synchronizes feeding/reward and metabolic processes. Rutter et al. 2002; Kennaway et al. 2007; and Hastings, 2007. Motivational state, hedonic signals and metabolic cues can reset SCN timing. Mendoza J, 2008, 2010; Kohsaka et al. 2007; and Lavialle et al. 2008. In rodents, loss of the daily dopamine peak at the SCN at the onset of locomotor activity is associated with insulin resistance/glucose intolerance and obesity. Reduction of dopamine at the SCN by 6-OHDA neurotoxin lesion induces glucose intolerance and obesity. Luo, 1997, Neuroreport, 8:3495.

As described in the present specification, the inventors have discovered that the activation of SuMN neurons with AMPA, a GABA_{A} antagonist, an NMDA receptor agonist or a blood-brain barrier penetrating dopamine analog improves dysmetabolism and that the activity of dopaminergic projections from the supramammillary nucleus (SuMN) to the SCN contribute to a circadian system for regulation of metabolism and energy balance and that stimulation of SuMN neurons with AMPA, a GABA_{A} antagonist, an NMDA receptor agonist or a blood-brain barrier penetrating dopamine analog in obese, insulin resistant animals reduced obesity, insulin resistance, glucose intolerance, and metabolic syndrome. These results demonstrate that the SuMN is a therapeutic target for treatment of a metabolic disorder or a key element thereof.

### SUMMARY

In one aspect, the invention provides a method of treating a metabolic disorder or a key element thereof by administering an agent that increases the firing rate of SuM neurons including those that release dopamine from a dopaminergic neuron in the supramammillary nucleus (SuMN) of a subject suffering from the metabolic disorder or a key element thereof.

In another aspect, the invention provides a method of treating a metabolic disorder or a key element thereof by administering to a subject with a metabolic disorder or a key element thereof an agent that increases dopamine release from a dopaminergic neuron in the SuMN of the subject and that projects into the SCN of the patient.

In another aspect, the invention provides a method of treating a metabolic disorder or a key element thereof by administering an AMPA receptor agonist to a patient suffering from a metabolic disorder or a key element thereof.

In another aspect, the invention provides a method of treating a metabolic disorder or a key element thereof by administering a GABA_{A} antagonist to a patient suffering from a metabolic disorder or a key element thereof.

In another aspect, the invention provides a method of treating a metabolic disorder or a key element thereof by administering an NMDA receptor agonist to a patient suffering from a metabolic disorder or a key element thereof.

In another aspect, the invention provides a method of treating a metabolic disorder or a key element thereof by administering a blood-brain barrier penetrating dopamine analog to a patient suffering from a metabolic disorder or a key element thereof.

In another aspect, the invention provides a method of treating a metabolic disorder or a key element thereof by administering, at a time of day when the dopamine level is high in a healthy individual, a blood-brain barrier penetrating dopamine analog to a patient suffering from a metabolic disorder or a key element thereof.

In another aspect, the invention provides a method of stimulating dopamine release from dopaminergic neurons in the SuMN of an individual by contacting the dopaminergic neurons with an AMPA receptor agonist, NMDA receptor agonist, or a GABA_{A} antagonist.

In another aspect, the invention provides a method of stimulating dopamine release from dopaminergic neurons in the SuMN of a subject by administering an AMPA receptor agonist, NMDA receptor agonist, or a GABA_{A} antagonist to the subject.

In another aspect, the invention provides a method of treating a metabolic disorder or a key element thereof by increasing the ratio of AMPA receptor or NMDA receptor activity to GABA-ergic receptor activity in neurons of the SuMN of an individual suffering from a metabolic disorder or a key element thereof.

In another aspect, the invention provides a method of treating a metabolic disorder or a key element thereof by administering to a patient an AMPA receptor agonist and/or an NMDA receptor agonist plus a GABA_{A} antagonist in amounts that together provide a therapeutically effective amount of active agents to treat a metabolic disorder or a key element thereof.

In another aspect, the invention provides a method of treating a metabolic disorder or a key element thereof by adjusting an aberrant daily rhythm of dopamine release in the SuMN of a patient to approach a normal daily rhythm of dopamine release in the SuMN of normal individuals of the same species and gender who not suffer from a metabolic disorder or a key element thereof.

In another aspect, the invention provides a method of normalizing a daily rhythm of dopamine release from a dopaminergic neuron in the SuMN by administering an AMPA receptor agonist, NMDA receptor agonist, a GABA_{A} antagonist to an individual suffering from an aberration of the daily rhythm.

In another aspect, the invention provides a method of normalizing the daily rhythm of dopamine at the SCN by administering a blood brain barrier penetrating dopamine analog to an individual suffering from an aberration of the daily rhythm.

In another aspect, the invention provides a method of treating a metabolic disorder or key element thereof by administering an AMPA receptor agonist, a GABA-ergic receptor antagonist, an NMDA receptor agonist or a blood brain barrier penetrating dopamine analog a to a subject suffering from the metabolic disorder or a key element thereof, to increase CNS dopamine activity at about the time of day of that is the time of day that dopamine activity peaks in the CNS of healthy individuals of the same species and gender.

In another aspect, the invention provides a pharmaceutical composition comprising an AMPA receptor agonist and/or an NMDA agonist, and/or a blood brain barrier penetrating dopamine analog, and/or a GABAA antagonist and a pharmaceutically acceptable excipient.

The details of one or more embodiments of the invention are set forth in the accompanying drawings and the description below. Other features, objects, and advantages of the invention will be apparent from the description and drawings, and from the items.

### BRIEF DESCRIPTION OF DRAWINGS

FIGS 1A-C shows results that demonstrated stimulation of SuMN neurons by AMPA infusion produced elevated dopamine metabolites HVA (1A) and DOPAC (1B) in SCN, but not serotonin metabolite 5-HIAA (1C).
FIG 2 shows results that demonstrated circadian rhythm of dopamine neuron activity in SuMN was dampened by high fat feeding.
FIGS 3A-E show results that demonstrated daily infusion of AMPA to SuMN reversed high fat diet-induced obesity and insulin resistance in high fat diet-resistant rats.
FIGS 4A-D show results that demonstrate infusion of AMPA into SuMN reduced glucose intolerance and insulin resistance in high fat diet sensitive rats. * = p < 0.05.
FIG 5 shows results that demonstrated GABA_{A} agonist/AMPA antagonist daily infusion induced glucose intolerance.
FIG 6 shows results that demonstrate that enhancing circadian dopamine transmission at the SuMN improves HFD-induced glucose intolerance/insulin resistance.
FIG 7 shows results that demonstrate that restoring circadian dopamine peak at the SCN improves HFD-induced glucose intolerance/insulin resistance.

### DETAILED DESCRIPTION

As described herein, experimental results demonstrate that SuMN dopamine neurons extend projections and release dopamine into the SCN. The observed release of dopamine from the SuMN neurons to the SCN, moreover, undergoes a daily rhythm, wherein a daily peak of dopaminergic activity occurs at about the onset of locomotor activity. The results reported herein demonstrate that a high fat diet reduces the daily peak of SuMN dopamine neuron activity, that activation of SuMN dopamine neurons reduced metabolic abnormalities induced by a high fat diet, whereas, in complementary fashion, inhibition of SuMN dopamine neurons induced metabolic disorders. The results described herein also suggest that the elevated release of dopamine from the SuMN neurons upon stimulation of AMPA receptor activity in the SuMN is at least partially responsible for positive metabolic effects of the AMPA agonist. Similar results have been obtained using NMDA receptor agonists applied to the SuMN.

One practical application of the results reported herein is that the SuMN is a therapeutic target for treatment of a metabolic disorder or a key element thereof.

### Methods of Use

Methods described herein include methods of treating metabolic disorders or a key element thereof in mammals including humans.

The term "metabolic disorder" refers to disorders associated with aberrant whole-body glucose, lipid and/or protein metabolism of a species and pathological consequences arising from such disorder. A metabolic disorder may or may not be associated with an aberrant patterns in the daily levels (and fluctuations) of a neuroendocrine transmitter, e.g., dopamine.

The "key elements" of metabolic disorders include but are not limited to, type 2 diabetes, pre-diabetes (impaired fasting glucose or impaired glucose tolerance), metabolic syndrome or indices (key elements) thereof (increased waist circumference, increased fasting plasma glucose, increased fasting plasma triglycerides, decreased fasting high density lipoprotein level, increased blood pressure), insulin resistance, hyperinsulinemia, cardiovascular disease (or key elements thereof such as arteriosclerosis, coronary artery disease, peripheral vascular disease, or cerebrovascular disease), congestive heart failure, obesity, fatty liver diseases, elevated plasma norepinephrine, elevated cardiovascular-related inflammatory factors, vascular endothelial dysfunction, elevated plasma factors potentiating vascular endothelial dysfunction, hyperlipoproteinemia, arteriosclerosis or atherosclerosis, hyperphagia, hyperglycemia, hyperlipidemia, and hypertension or high blood pressure, increased plasma postprandial triglyceride or free fatty acid levels, increased cellular oxidative stress or plasma indicators thereof, increased circulating hypercoagulative state, renal disease including renal failure and renal insufficiency.

In a method of the invention, a subject suffering from a metabolic disorder or a key element thereof is treated by administering an agent that increases dopamine release from a dopaminergic neuron in the supramammillary nucleus (SuMN) or increases dopamine levels at the SCN. Without being bound by theory, in some aspects, the agent contacts neurons in the SuMN. Furthermore, the agent contacts dopaminergic neurons within the SuMN, some of which project to the SCN area and release dopamine into the SCN. The dopamine released into the SCN can cause the daily rhythm in the SCN of the subject to more closely resemble the daily rhythm of a normal, healthy individual(not afflicted with a metabolic disorder or key element thereof) of the same species and gender as the subject.

Certain methods described herein relate to adjusting an aberrant daily rhythm of dopamine release in the SuMN of a subject suffering from a metabolic disorder or a key element thereof to approach a normal daily rhythm of dopamine release by a dopaminergic neuron in the SuMN of normal individuals of the same species and gender as the subject. Such adjustment is accomplished by adjusting a daily rhythm of dopamine release in the SuMN to exhibit the normal peak that occurs at or around the time of onset in locomotory activity. In diurnal animals, e.g., humans, the onset of locomotor activity is typically at or around the time of awakening. Methods directed to adjusting an aberrant daily rhythm of dopamine release in the SuMN in diurnal animals may therefore be accomplished by administering an agent at a predetermined time, such that the effect of the agent on dopamine release in the SuMN is effected at about the time of the onset of locomotor activity. In diurnal animals, for example humans, an immediate onset formulation of an active agent may be administered at or around the time of awakening, e.g., within about two hours of awakening. A delayed onset formulation of an active agent may, for example, be administered to a human before bedtime.

Certain methods described herein relate to increasing the ratio of AMPA receptor activity to gabanergic receptor activity in the SuMN. Such a ratio may be increased thus increasing AMPA receptor activity in the SuMN, decreasing gabanergic receptor activity in the SuMN, or by both increased increasing AMPA receptor activity in the SuMN and decreasing gabanergic receptor activity in the SuMN. Agents that increase AMPA receptor activity in the SuMN include AMPA receptor agonists and NMDA receptor agonists. Agents that decrease gabanergic receptor activity in the SuMN include gabanergic receptor antagonists, e.g., GABA_{A} antagonists.

Certain methods described herein relate to a method of treating a metabolic disorder or a key element thereof by administering an AMPA receptor agonist or an NMDA recptor agonist to a subject in need of treatment of the metabolic disorder or a key element thereof.

Certain methods described herein relate to a method of treating a metabolic disorder or a key element thereof by administering an AMPA receptor agonist or an NMDA receptor agonist to a subject in need of treatment of the metabolic disorder or a key element thereof.

Certain methods described herein relate to a method of treating a metabolic disorder or key element thereof by administering an AMPA receptor agonist, an NMDA receptor agonist, or a gabanergic receptor antagonist to a subject suffering from the metabolic disorder or a key element thereof, to increase CNS dopamine activity at about the time of day of that is the time of day that dopamine peaks in the CNS of healthy individuals of the same species and gender.

The methods described herein may be performed in subjects, individuals or patients in which such methods are desirable. A subject, individual or patient, for example, may suffer from a condition in need of treatment. Such conditions in need of treatment include the metabolic disorders and key elements thereof disclosed herein. A preferred subject, individual or patient for the methods described herein is a human, and includes male and female humans.

In preferred aspects, the methods described herein include, without limitation, treatment of type 2 diabetes, pre-diabetes, cardiovascular disease, metabolic syndrome, or obesity.

The methods described herein may be performed with a single active agent, e.g., as monotherapy. Alternatively, the methods described herein may be performed with a combination of two active agents or alternatively a combination of more than two active agents. A preferred combination of active agents for performing the methods described herein is a combination of an agent that increases AMPA receptor activity or NMDA receptor activity in the SuMN and an agent the decreases gabanergic receptor activity in the SuMN. An example of such a combination is a combination comprising an AMPA receptor agonist and a GABA_{A} antagonist. NMDA receptor agonists also increase dopamine release from dopaminergic neurons in the supramammillary nucleus and can be employed in combination with a GABA_{A} antagonist to treat metabolic disorders or key elements of such disorders.

### Modification of Neuroendocrine Daily Rhythms

Healthy (normal) subjects, i.e., lean members of a species not suffering from such metabolic disorder and/or key elements thereof have highly predictable daily neuroendocrine release profiles. As disclosed herein, release of dopamine from the SuMN neurons to the SCN undergoes a daily rhythm, exhibiting a daily peak of dopaminergic activity that occurs at about the onset of locomotor activity. Metabolic disorders or a key element thereof can be treated by modifying an aberrant daily rhythm of dopamine release from neurons in the SuMN so that the daily rhythm resembles, or more closely approximates in phase and amplitude, the normal diurnal plasma rhythm of lean, young and healthy members of the same species and sex. Such modulation of dopamine release from neurons in the SuMN can be used to treat metabolic syndromes such as for example and without limitation, type 2 diabetes, obesity, insulin resistance, hyperinsulinemia hyperglycemia, hyperlipoproteinemia, hyperphagia, prediabetes, metabolic syndrome, cardiovascular diseases, obesity, insulin resistance (impaired glucose tolerance), hyperlipidemia, fatty liver diseases, etc.

### Stimulation of SuMN Activity

In certain aspects, the application discloses stimulation of SuMN activity. Activity in SuMN may be stimulated, for example, by increasing, i.e., stimulating, the activity of dopamine neurons in the SuMN. At least some SuMN dopamine neurons extend and release dopamine into the SCN. Stimulation of SuMN activity, e.g., stimulation of SuMN dopamine neurons, can therefore be used to increase dopamine in the SCN.

### Compounds for Use in Disclosed Methods

Compounds for use in the methods disclosed herein modify CNS neuronal activity. Examples of modifying CNS neuronal activity include stimulating activity in the SuMN or increasing the levels of dopamine at the SCN.

Compounds for modifying CNS neuronal activity include the following non-limiting classes and examples.

### AMPA Receptor Agonists

Non-limiting examples of AMPA receptor agonists that may be used in the methods disclosed herein include, e.g., (RS)-α-Amino-3-hydroxy-5-methyl-4-is-oxazolepropionic acid hydrobromide ("(RS)-AMPA hydrobromide"); (R)-α-Amino-3-hydroxy-5-methyl-4-iso-xazolepropionic acid ("(R)-AMPA"); (RS)-α-Amino-3-hydroxy-5-methyl-4-is-oxazolepropionic acid ("(RS)-AMPA"); (S)-α-Amino-3-hydroxy-5-methyl-4-iso-xazolepropionic acid ("(S)-AMPA"); RS)-2-Amino-3-(4-chloro-3-hydroxy--5-isoxazolyl)propionic acid ("Cl-HIBO"); (S)-α-Amino-2,3,4,5,6,7-hexahydro-2,-4-dioxo-1H-cyclopentapyrimidine-1-propanoic acid ("(S)-CPW 399"); (S)-(-)-α-Amino-5-fluoro-3,4-dihydro--2,4-dioxo-1(2H)pyridinepropanoic acid ("(S)-(-)-5-Fluorowillardiine"); NPEC-caged-(S)-AMPA ("(N)-1-(2-Nitrophenyl)ethylcarboxy-(-S)-α-1-(2-nitrophenyl)ethylcarboxyamino-3-hydroxy-5--methyl-4-isoxazolepropionic acid"); and (L)-(+)-α-Amino-3,5-dioxo-1,2,4-oxad-iazolidine-2-propanoic acid ("L-Quisqualic acid") (available from Tocris Bioscience (Bristol, UK) and R&D Systems Inc. (Minneapolis, MN)); (S)-2-amino-3-(3-hydroxy-5-phenylisoxazol-4-yl)propionic acid ((S)-APPA), 2-pyridyl analogue of APPA, (RS)-2-amino-3-[3-hydroxy-5-(2-pyridyl)isoxazol-4-yl]propionic acid (2-Py-AMPA) (*see*, e.g., Johansen TN, et al. Chirality 1997;9(3):274-80); CIP-AS (*see*, Conti, P. et al. J Med Chem 1999;42(20):4099-4107); 5-HPCA and 7-HPCA (*see*, Hansen, J. et al. "AMPA receptor agonists: structural, conformational and stereochemical aspects"; Excitatory Amino Acid Receptors: Design of Agonists and Antagonists; 1992 Ellis Horwood Limited, Chichester, West Sussex, England; p. 216); N,N'-(2,2'-(1,4-phenylene)bis(ethane-2,1 -diyl))dipropane-2-sulfonamide; and AMPA Activator (CMPA) (available from EMD Millipore (Billerica, MA)).

### AMPA Receptor Potentiators

It is known that the rapid desensitization and deactivation of AMPA receptors to glutamate may be inhibited using certain compounds. This action of these compounds is often referred to as "potentiation" of the receptors. One such compound, which selectively potentiates AMPA receptor function, is cyclothiazide. Partin et al., Neuron. Vol. 11, 1069-1082, 1993. Compounds which potentiate AMPA receptors, like cyclothiazide, are often referred to as ampakines. Ampakines are drugs developed using aniracetam as a structural antecedent. Aniracetam was found to have nootropic effects in whole animals, attributed to its anti-deactivation/anti-desensitization activities at the AMPA receptor. Ampakines bind to AMPA receptors and prevent or reverse AMPA receptor deactivation and desensitization. These drugs potentiate AMPA-type ionotropic glutamate receptor currents. Potentially two ampakine formulations, CX546 and CX717, reverse respiratory depression due to opiate intoxication (Funk, G. D. and Greer, J. J. (2006) Am. J. Respir. Crit. Care Med. 174:1384-91; Ren J., et al. (2009) Anesthesiology. 110(6):1364-70), while CX546, may ameliorate the pathological respiratory symptoms of Rett Syndrome (Ogier, M., et al. (2007) J Neurosci, 27:10912-17).

A different class of drugs, the benzothiadiazides, also has some of the same pharmacological properties at AMPA receptors. Benzothiadiazide diuretics were the first drugs used for single-agent management of mild to moderate hypertension. Along with β-blockers and angiotensin-converting enzyme inhibitors, these diuretics served as a primary treatment option for hypertension. Benzothiadiazides also reverse AMPA receptor desensitization though allosteric modulation. This, however, is not the only function of these agents in the nervous system. For example, cyclothiazide also inhibits γ-aminobutyric acid Type A (GABA_{A}) and glycine receptors, thus reducing inhibition. Additionally, these drugs modulate presynaptic release at fast excitatory synapses and inhibitory synapses. Non-limiting examples of benzothiadiazide compounds suitable for use in the practice of the embodiments described here are disclosed in PCT Int'l Pub. No. WO 9812185 and PCT Int'l Pub. No. WO 9942456.

Certain substituted 2.1.3 benzoxadiazole compounds have been found to be significantly and surprisingly more potent in animal models of attention deficit hyperactivity disorder (ADHD), schizophrenia and cognition than previously disclosed compounds in US 2002/0055508 and US 2002/0099050. This new class of N,N-disubstituted amides (I) display significant activity for enhancing AMPA mediated glutamateric synaptic responses.

Thus, also contemplated for use herein are AMPA receptor potentiators, including, but not limited to, pyrroliddones (e.g., piracetam, aniracetam), benzothiazides (e.g., cyclothiazide), benzylpiperidines (e.g., CX-516 (Ampalex), CX-546, CX-717, CX-1739); biarylpropylsulfonamides (e.g., LY392098, LY404187 and LY503430) (see, O'Neill, M.J. et al. Curr Drug Targets CNS Neurol Disord. 2004 Jun;3(3):181-94); and 2-oxo-1-pyrrolidineacetamide (Trade names: Breinox, Dinagen, Lucetam, Nootropil, Nootropyl, Oikamid).

Additional non-limiting examples of AMPA receptor modulators suitable for use in the practice of the presently described methods are disclosed in PCT Int'l Pub. No. WO 9402475 and in related U.S. Pat. Nos. 5,773,434; 5,488,049; 5,650,409; 5,736,543; 5,747,492; 5,773,434; 5,891,876; 6,030,968; 6,274,600; 6,329,368; 6,943,159; 7,026,475; and U.S. Pat. Pub. No. 20020055508. Further non-limiting examples of AMPA receptor modulators suitable for use in the methods disclosed herein include: sulfonamide derivatives as disclosed in U.S. Pat. Nos. 6,174,922; 6,303,816; 6,358,981; 6,362,230; 6,500,865; 6,515,026; 6,552,086; PCT Int'l Pub. Nos. WO 0190057, WO 0190056, WO 0168592, WO 0196289, WO 02098846, WO 0006157, WO 9833496, WO 0006083, WO 0006148, WO 0006149, WO 9943285, WO 9833496; (bis)sulfonamide derivatives as disclosed in WO 0194306; N-substituted sulfonamide derivatives as disclosed in U.S. Pat. No. 6,525,099 and PCT Int'l Pub. No. WO 0006537; heterocyclic sulfonamide derivatives as disclosed in U.S. Pat. No. 6,355,655 and PCT Int'l Pub. Nos. WO 0214294, WO 0214275, and WO 0006159; heterocyclyl sulfonamide derivatives as disclosed in U.S. Pat. No. 6,358,982 and PCT Int'l Pub. No. WO 0006158; alkenyl sulfonamide derivatives as disclosed in U.S. Pat. No. 6,387,954 and PCT Int'l Pub. No. WO 0006539; cycloalkenyl sulfonamide derivatives as disclosed in PCT Int'l Pub. No. WO 02098847; cyclopentyl sulfonamide derivatives as disclosed in U.S. Pat. No. 6,639,107 and PCT Int'l Pub. No. WO 0142203; cycloalkylfluoro sulfonamide derivatives as disclosed in PCT Int'l Pub. No. WO 0232858; acetylenic sulfonamide derivatives as disclosed in PCT Int'l Pub. No. WO0218329; 2-propane-sulfonamide compounds and derivatives as disclosed in U.S. Pat. No. 6,596,716 and PCT Int'l Pub. Nos: WO 06087169, WO 06015827, WO 06015828, WO 06015829, WO 07090840, and WO 07090841; and 2-aminobenzenesulfonamide derivatives as disclosed in WO 02089734.

Further non-limiting examples of AMPA receptor modulators suitable for use in the methods disclosed herein include: benzoyl piperidine, benzoyl derivatives, and pyrrolidine compounds and related structures as disclosed in U.S. Pat. Nos. 5,650,409; 5,747,492; 5,783,587; 5,852,008; and 6,274,600; compounds based on benzoxazine ring systems as disclosed in U.S. Pat. Nos. 5,736,543; 5,962,447; 5,985,871; and PCT Int'l Pub. Nos. WO 9736907 and WO 9933469; acylbenzoxazines as disclosed in U.S. Pat. No. 6,124,278, and PCT Int'l Pub. No. WO 9951240; carbonylbenzoxazine compounds as disclosed in PCT Int'l Pub. No. WO 03045315; substituted 2,3-benzodiazepin-4-ones as disclosed in U.S. Pat. No. 5,891,871; and benzofurazan compounds as disclosed in U.S. Pat. Nos. 6,110,935; 6,313,115; and PCT Int'l Pub. No. WO 9835950. Examples of benzofurazan compounds include 1-(benzofurazan-5-ylcarbonyl)-4,4-difluoropiperidine and 4-(benzofurazan-5-ylcarbonyl)morpholine. Substituted 5-oxo-5,6,7,8-tetrahydro-4H-1-benzopyrans and benzothiopyrans and related compounds as disclosed in PCT Int'l Pub. No. WO 0075123 are suitable for use as AMPA receptor modulators.

Additional AMPA receptor modulators suitable for use in the methods disclosed herein include amidophosphate derivatives as disclosed in U.S. Pat. No. 6,521,605, and PCT Int'l Pub. No. WO 0006176; monofluoralkyl derivatives as disclosed in PCT Int'l Pub. No. WO 0066546; and substituted quinazolines and analogs thereof as disclosed in PCT Int'l Pub. No. WO 9944612 and quainoxaline compounds and derivatives as disclosed in PCT Int'l Pub. No. WO 07060144. Additional compounds suitable for use as AMPA receptor modulators with practice of the embodiments include 2-ethoxy-4'-[3-(propane-2-sulfonylamino)-thiophen-2-yl]-biphenyl-4-carboxylic acid and derivatives thereof as disclosed in U.S. Pat. Pub. No. 20060276532; pyrrole and pyrazole compounds and derivatives thereof as disclosed in U.S. Pat. Pub. No. 20070066573; and the thiadiazine compounds and derivatives as disclosed in U.S. Pat. Pub. No. 20070004709; and the benzoxazepine compounds and derivatives as disclosed in U.S. Pat. Pub. No. 20040171605. Other compounds suitable for use as AMPA receptor modulators are disclosed in PCT Int'l Pub. Nos. WO 9942456, WO 0006156, WO 0157045, and U.S. Pat. No. 6,617,351.

### GABA Receptor Agonists

Non-limiting examples of GABA receptor signaling inhibitors (e.g., antagonists and reverse agonists), include metrazol; Flumazenil; (-)-alpha-Thujone, (1S,4R)-1-Isopropyl-4-methylbicyclo[3.1.0]hexan-3-one ("thujone") (Sigma-Aldrich); Gabazine; RU5135; 4-(3-biphenyl-5-(4-piperidyl)-3-isoxazole, and bilobalide (see Johnston, G. BJP Volume 169, Issue 2, pages 328-336, May 2013); SR95531 (see, Wall, M.J. Neuropharmacology Volume 44, Issue 1, January 2003, Pages 56-69). Commercially available GABA receptor antagonists that may be used in the methods disclosed herein include, e.g., [R-(R*,S*)]-5-(6,8-Dihydro-8-oxofur-o[3,4-e]-1,3-benzodioxol-6-yl)-5,6,7,8-tetrahydro--6,6-dimethyl-1,3-dioxolo[4,5-g]isoquinolinium iodide "((-)-Bicuculline methiodide"); [R-(R*,S*)]-5-(6,8-Dihydro-8-oxofur-o[3,4-e]-1,3-benzodioxol-6-yl)-5,6,7,8-tetrahydro--6,6-dimethyl-1,3-dioxolo[4,5-g]isoquinolinium bromide ("(-)-Bicuculline methobromide"); [R-(R*,S*)]-5-(6,8-Dihydro-8-oxofur-o[3,4-e]-1,3-benzodioxol-6-yl)-5,6,7,8-tetrahydro--6,6-dimethyl-1,3-dioxolo[4,5-g]isoquinolinium chloride ("(-)-Bicuculline methochloride"); [R-(R*,S*)]-6-(5,6,7,8-Tetrahydro-6--methyl-1,3-dioxolo[4,5-g]isoquinolin-5-yl)furo[3,-4-e]-1,3-benzodioxol-8(6H)-one ("(+)-Bicuculline"); 5-(Aminosulfonyl)-4-chloro-2-([2-fu-ranylmethyl]amino)benzoic acid ("Furosemide"); 4-(5-[1,1'-Biphenyl]-3-yl-1-hydroxy--1H-pyrazol-4-yl)piperidine trifluoroacetate ("PHP 501 trifluoroacetate"); Picrotoxinin; Salicylidene salicylhydrazide ("SCS"); 6,7-Dihydro-3-[(2-hydroxyethyl)thio-]-6,6-dimethyl-1-(2-thiazolyl)-benzo[c]thiophen-4(-5H)-one ("TB 21007"); and 4,5-Dihydro-4,4-dimethyl-imidazo[1,-5-a]quinoxaline-3-carboxylic acid 1,1-dimethylethyl ester ("U 93631") (Tocris Bioscience (Bristol, UK)).

Other non-limiting examples of GABA receptor signaling inhibitors (e.g., antagonists and reverse agonists) that can be used in the methods disclosed herein include, e.g., CGP36742, CGP56433, CGP56999, 5-aminovaleric acid HCl, 2-hydroxysaclofen, phaclofen, 2-(3-carboxypropyl)-3-amino-6-(4-methoxyphenyl)pyridazinium bromide; and those described in U.S. Pat. Pub. No. 2011/0224278 (see, e.g., Table 1).

The contents of any of the above patent applications, and in particular the general formulae of the therapeutically active compounds of the items and exemplified compounds therein, are incorporated herein in their entirety by reference thereto.

### NMDA Receptor Agonists

Non limiting examples of NMDA receptor agonists include cis-ACPD ((±)-1-Aminocyclopentane-*cis*-1,3-dicarboxylic acid), D-Aspartic acid, L-Aspartic acid, L- Cysteinesulfinic acid, GLYX 13 (Thr-Pro-Pro-Thr-NH₂), (R)-(+)-HA-996 ((R)-(+) 3-Amino-1-hydroxypyrrolidin, Homoquinolinic acid, Ibotenic acid, Spermidine trihydrochloride, (RS)-(Tetrazol-5-yl)glycine, L-Homocysteic acid, L-Cysteine hydrochloride , D-Cysteine hydrochloride, D-Cycloserine powder.

### Blood Brain Barrier Penetrating Dopamine analogs

Dopamine may be transported across the blood brain barrier by any of a variety of known carrier mechanisms for achieving such a result, including but not limited to, dopamine associated with nanoparticles of lipid, carbohydrate, and/or protein, dopamine associated with liposomes, and dopamine associated with cyclodextrins. Also, dopamine may be transported across the blood brain barrier as an ester of varying length that then is acted upon by esterases within the brain to release the free dopamine.

Non-limiting examples of blood brain barrier penetrating dopamine analogs useful in the methods of treatment described herein are disclosed in US Patents 6,297,226 , 8,697,898, 8,399,513, 8,324,273, 8,324272, 8,163,958, 7,956,212, 4,933,324, 4,939,174, 5,994,392, 6,107,489, 6,258,836, and 6,407,137.

US Patents 5,472,954 and 5,324,718 and 5,017,566 describe the use of cyclodextrins to carry dopamine across the blood brain barrier.

The disclosures of all of the above patents are incorporated herein by reference in their entirety.

Also within the scope of the invention are so-called "prodrugs" of compounds suitable for use in the present invention. Thus certain derivatives of compounds suitable for use in the present invention which may have little or no pharmacological activity themselves can, when administered into or onto the body, be converted into compounds having the desired activity, for example, by hydrolytic cleavage. Such derivatives are referred to as "prodrugs." Further information on the use of prodrugs may be found in Pro-drugs as Novel Delivery Systems, Vol. 14, ACS Symposium Series (T Higuchi and W Stella) and 'Bioreversible Carriers in Drug Design', Pergamon Press, 1987 (ed. E B Roche, American Pharmaceutical Association).

Prodrugs in accordance with the invention can, for example, be produced by replacing appropriate functionalities present in the compounds suitable for use in the present invention with certain moieties known to those skilled in the art as "pro-moieties," as described, for example, in "Design of Prodrugs" by H Bundgaard (Elsevier, 1985).

Certain compounds suitable for use in the present invention may themselves act as prodrugs of other compounds suitable for use in the present invention.

In another embodiment, the present invention provides pharmaceutical compositions comprising compounds having the same type of activity, enantiomers, diastereomers, N-oxides, crystalline forms, hydrates, solvates or pharmaceutically acceptable salts of such compounds, in admixture with pharmaceutically acceptable diluents or carriers such as those disclosed.

A metabolite of a compound disclosed herein is a derivative of a compound which is formed when the compound is metabolized. The term "active metabolite" refers to a biologically active derivative of a compound which is formed when the compound is metabolized.

The term "metabolized" refers to the sum of the processes by which a particular substance is changed in the living body. In brief, all compounds present in the body are manipulated by enzymes within the body in order to derive energy and/or to remove them from the body. Specific enzymes produce specific structural alterations to the compound. For example, cytochrome P450 catalyzes a variety of oxidative and reductive reactions while uridine diphosphate glucuronyltransferases catalyze the transfer of an activated glucuronic-acid molecule to aromatic alcohols, aliphatic alcohols, carboxylic acids, amines and free sulphydryl groups. Further information on metabolism may be obtained from The Pharmacological Basis of Therapeutics, 9th Edition, McGraw-Hill (1996), pages 11-17.

Metabolites of the compounds disclosed herein can be identified either by administration of compounds to a host and analysis of tissue samples from the host, or by incubation of compounds with hepatic cells in vitro and analysis of the resulting compounds. Both methods are well known in the art.

### Pharmaceutical Compositions

The invention provides pharmaceutical compositions comprising one or more compounds, or a crystalline form, hydrate, solvate, active metabolite or pharmaceutically acceptable salt of such compounds for use in the methods described herein.

A pharmaceutical composition may also include optional additives, such as a pharmaceutically acceptable carrier or diluent, a flavoring, a sweetener, a preservative, a dye, a binder, a suspending agent, a dispersing agent, a colorant, a disintegrator, an excipient, a diluent, a lubricant, an absorption enhancer, a bactericide and the like, a stabilizer, a plasticizer, an edible oil, or any combination of two or more of said additives.

Suitable pharmaceutically acceptable carriers or diluents include, but are not limited to, ethanol, water, glycerol, aloe vera gel, allantoin, glycerine, vitamin-A and E oils, mineral oil, phosphate buffered saline, PPG2 myristyl propionate, magnesium carbonate, potassium phosphate, vegetable oil, animal oil and solketal.

Suitable binders include, but are not limited to, starch, gelatin, natural sugars such as glucose, sucrose and lactose, corn sweeteners, natural and synthetic gums such as acacia, tragacanth, vegetable gum, sodium alginate, carboxymethylcellulose, polyethylene glycol, waxes and the like.

Suitable disintegrators include, but are not limited to, starch such as corn starch, methyl cellulose, agar, bentonite, xanthan gum and the like.

Suitable lubricants include, but are not limited to, sodium oleate, sodium stearate, magnesium stearate, sodium benzoate, sodium acetate, sodium chloride and the like.

Suitable suspending agents include, but are not limited to, bentonite.

Suitable dispersing and suspending agents include, but are not limited to, synthetic and natural gums such as vegetable gum, tragacanth, acacia, alginate, dextran, sodium carboxymethylcellulose, methylcellulose, polyvinylpyrrolidone and gelatin.

Suitable edible oils include, but are not limited to, cottonseed oil, sesame oil, coconut oil and peanut oil.

Examples of additional additives include, but are not limited to, sorbitol, talc, stearic acid and dicalcium phosphate.

In certain embodiments, the invention provides pharmaceutical compositions comprising an AMPA receptor agonist and/or an NMDA receptor agonist and/or a GABA_{A} antagonist and a pharmaceutically acceptable excipient, more preferably, such composition comprises a first amount of said AMPA receptor agonist and/or NMDA receptor agonist plus a second amount of said GABA_{A} antagonist that provide a synergistic effect in treatment a metabolic disorder or key element thereof when administered to an individual in need of treatment. More preferably, such compositions comprise a first amount of an AMPA receptor agonist that is below an effective amount of said AMPA receptor agonist to treat the metabolic disorder or key element thereof, and/or an NMDA receptor agonist, for monotherapy treatment of metabolic disorder or key element thereof and/or a first amount of GABA_{A} antagonist that is below an effective amount of GABA_{A} antagonist for monotherapy treatment of said metabolic disorder or key element thereof. Most preferably, such compositions comprise a first amount of AMPA receptor agonist and/or NMDA receptor agonist that is below an effective amount of AMPA receptor agonist and/or NMDA receptor agonist for monotherapy treatment of a metabolic disorder or key element thereof and a second amount of GABA_{A} antagonist that is below an effective amount of GABA_{A} antagonist for monotherapy treatment of metabolic disorder or key element thereof. An NMDA receptpr agonist may be used to supplement or in place of an AMPA receptor agonist.

As used herein, a "synergistic effect" refers to a superadditive effect obtained when agents are administered in combination, compared to when such agents are administered individually.

### Unit Dosage Forms

The pharmaceutical composition may be formulated as unit dosage forms, such as tablets, pills, capsules, boluses, powders, granules, sterile parenteral solutions, sterile parenteral suspensions, sterile parenteral emulsions, elixirs, tinctures, metered aerosol or liquid sprays, drops, ampoules, autoinjector devices or suppositories. The unit dosage forms may be used for oral, parenteral, intranasal, sublingual or rectal administration, or for administration by inhalation or insufflation, transdermal patches, and a lyophilized composition. In general, any delivery of active ingredients that results in systemic availability of such ingredients can be used. Preferably the unit dosage form is an oral dosage form, most preferably a solid oral dosage; therefore the preferred dosage forms are tablets, pills and capsules. However, parenteral preparations, i.e., preparations wherein active agent is not substantially absorbed through the gut, are preferred too.

Solid unit dosage forms may be prepared by mixing the active agents of the present invention with a pharmaceutically acceptable carrier and any other desired additives as described above. The mixture is typically mixed until a homogeneous mixture of the active agents of the present invention is obtained and the carrier and any other desired additives are formed, i.e., the active agents are dispersed evenly throughout the composition. In this case, the composition can be formed as dry or moist granules.

Tablets or pills can be coated or otherwise prepared so as to form a unit dosage form that has delayed and/or sustained action, such as controlled release and delayed release unit dosage forms. For example, the tablet or pill can comprise an inner dosage and an outer dosage component, the latter being in the form of a layer or envelope over the former. The two components can be separated by an enteric layer which serves to resist disintegration in the stomach and permits the inner component to pass intact into the duodenum or to be delayed in release.

Biodegradable polymers for controlling the release of the active agents include, but are not limited to, polylactic acid, polyepsilon caprolactone, polyhydroxybutyric acid, polyorthoesters, polyacetals, polydihydropyrans, polycyanoacrylates and crosslinked or amphipathic block copolymers of hydrogels.

For liquid dosage forms, the active substances or their physiologically acceptable salts are dissolved, suspended or emulsified, optionally with the usually employed substances such as solubilizers, emulsifiers or other auxiliaries. Solvents for the active combinations and the corresponding physiologically acceptable salts can include water, physiological salt solutions or alcohols, e.g., ethanol, propanediol or glycerol. Additionally, sugar solutions such as glucose or mannitol solutions may be used. A mixture of the various solvents mentioned may be used in the present invention too.

A transdermal dosage form is contemplated by the present invention too. Transdermal forms may be a diffusion transdermal system (transdermal patch) using either a fluid reservoir or a drug-in-adhesive matrix system. Other transdermal dosage forms include, but are not limited to, topical gels, lotions, ointments, transmucosal systems and devices, and iontophoretic (electrical diffusion) delivery systems. Transdermal dosage forms may be used for delayed release and sustained release of the active agents of the present invention.

The pharmaceutical compositions and unit dosage forms of the present invention for parenteral administration, and in particular by injection, typically include a pharmaceutically acceptable carrier, as described above. A preferred liquid carrier is vegetable oil. Injection may be, for example, intravenous, epidural, intrathecal, intramuscular, intraluminal, intratracheal or subcutaneous.

The active agents can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active agents of the present invention may also be coupled with soluble polymers such as targetable drug carriers. Such polymers include, but are not limited to, polyvinylpyrrolidone, pyran copolymers, polyhydroxypropylmethacrylamidophenol, polyhydroxyethylaspartamidophenol, and polyethylenoxypolylysine substituted with palmitoyl residues.

### Administration

The pharmaceutical composition or unit dosage forms of the present invention may be administered by a variety of routes, such as the oral and enteral, intravenous, intramuscular subcutaneous, transdermal, transmucosal (including rectal and buccal) and by inhalation routes. Preferably, the oral or transmucosal route is used (i.e., with solid or liquid formulations or skin patches, respectively).

### Dosage Amounts

As used herein, the term "effective amount" refers to an amount that results in measurable amelioration of at least one symptom or parameter of a specific disorder.

The pharmaceutical composition or unit dosage form of the present invention may be administered according to a dosage and administration regimen defined by routine testing in light of the guidelines given above in order to obtain optimal activity while minimizing toxicity or side effects for a particular patient. However, such fine tuning of the therapeutic regimen is routine in the light of the guidelines given herein.

The dosage of the active agents of the present invention may vary according to a variety of factors such as underlying disease conditions, the individual's condition, weight, sex and age, and the mode of administration. An effective amount for treating a disorder can easily be determined by empirical methods known to those of ordinary skill in the art, for example by establishing a matrix of dosages and frequencies of administration and comparing a group of experimental units or subjects at each point in the matrix. The exact amount to be administered to a patient will vary depending on the state and severity of the disorder and the physical condition of the patient. A measurable amelioration of any symptom or parameter can be determined by a person skilled in the art or reported by the patient to the physician. It will be understood that any clinically or statistically significant attenuation or amelioration of any symptom or parameter of a metabolic disorder or key element thereof is within the scope of the invention. Clinically significant attenuation or amelioration means perceptible to the patient and/or to the physician.

Drug combinations of, e.g., are not only highly effective at relatively low doses but also possesses low toxicity and produces few side effects. The amount of an active agent to be administered can typically range between about 0.01 and about 50 mg/kg/day, preferably between about 0.1 and about 25 mg/kg/day and more preferably between 0.2 and about 12 mg/kg/day. Total amount of active agent administered daily can typically range between about 0.75 and about 3750 mg, preferably between about 7.5 and about 1875 mg, and more preferably between about 15 and about 900 mg. It will be understood that the pharmaceutical formulations of the present invention need not necessarily contain the entire amount of the agent that is effective in treating the disorder, as such effective amounts can be reached by administration of a plurality of doses of such pharmaceutical formulations.

In a preferred embodiment of the present invention, the compounds are formulated in capsules or tablets, preferably containing 50 to 200 mg of the compounds of the invention, and are preferably administered to a patient at a total daily dose of 50 to 400 mg, preferably 150 to 250 mg and most preferably about 200 mg, for treatment, for example of a metabolic disorder or key element thereof, e.g. type 2 diabetes.

A pharmaceutical composition for parenteral administration contains from about 0.01% to about 100% by weight of the active agents of the present invention, based upon 100% weight of total pharmaceutical composition.

Generally, transdermal dosage forms contain from about 0.01% to about 100% by weight of the active agents versus 100% total weight of the dosage form.

### Combination Therapy

As used herein, the term "combination" used in conjunction with the terms therapy, treatment, or administration refers to therapy, treatment or administration of, or with a first amount of a first active agent, e.g., an AMPA receptor agonist, , or an NMDA receptor agonist, and a second amount of a second active agent, e.g., a GABA_{A} antagonist, wherein the first and second amounts together comprise a therapeutically effective amount to treat a metabolic disorder of key element thereon subject in need of treatment. The term combination encompasses administration of first and second amounts of compounds in an essentially concomitant manner, such as in a single pharmaceutical composition, for example, capsule or tablet having a fixed ratio of first and second amounts, or in multiple, separate capsules or tablets for each. The term combination also encompasses administration of first and second amounts of compound in a sequential manner, in either order. When combination treatment involves the sequential administration of the first amount of, e.g., AMPA receptor agonist and the second amount of, e.g., a GABA_{A} antagonist, the compounds are administered sufficiently close in time to have the desired therapeutic effect. For example, the period of time between each administration which can result in the desired therapeutic effect, can range from minutes to hours and can be determined taking into account the properties of each compound such as potency, solubility, bioavailability, plasma half-life and kinetic profile. For example, agents can be administered in any order within about 16 hours of each other, within about 8 hours of each other, within about 4 hours of each other, within about 1 hour of each other, within about 30 minutes of each other or within about 10 minutes of each other.

A pharmaceutical composition or unit dosage form may be administered in a single daily dose, or the total daily dosage may be administered in divided doses. In addition, simultaneous or sequential administration of another compound for the treatment of the disorder may be desirable. The order of administration will depend upon a variety of factors including age, weight, sex and medical condition of the patient; the severity and etiology of the disorders to be treated, the route of administration, the renal and hepatic function of the patient, the treatment history of the patient, and the responsiveness of the patient. Determination of the order of administration may be fine-tuned and such fine-tuning is routine in the light of the guidelines given herein.

### Packaged Kits

In another embodiment, a packaged kit is provided that contains one or more pharmaceutical formulations to be administered, i.e., one or more pharmaceutical formulations containing an active agent to be administered in combination to treat a metabolic disorder or key element thereof, a container, preferably sealed, for housing the one or more formulations during storage and prior to use, and instructions for carrying out drug administration in a manner effective to treat to a metabolic disorder or key element thereof in a patient. The instructions will typically be written instructions on a package insert and/or on a label. Depending on the type of formulation and the intended mode of administration, the kit may also include a device for administering the formulation. The formulation may be any suitable formulation as described herein. For example, the formulation may be an oral dosage form containing a unit dosage of selected active agents. The kit may contain multiple formulations of different dosages of the same agent.

The kit may also contain multiple formulations of different active agents. If more than one formulation comprising is present, for example in order to provide for repeat dosing, such formulations may be the same, or may be different in terms of the dosage, chemical composition and/or physical form.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended embodiments. Although specific terms are employed herein, they are used in a generic and descriptive sense only and not for purposes of limitation.

Unless context dictates otherwise, the ratios of compounds set forth herein refer to relative amounts on a weight to weight basis (w/w).

The invention is further illustrated by the following examples.

### Example 1: Dopamine Neurons in SuMN Project to and release dopamine at the SCN

### Protocols

Female Sprague-Dawley rats (12) were housed in a room at constant temperature and humidity with a 14/10 light/dark cycle were fed regular diet ad libitum. Each rat was microinjected with retrograde tracer fluorogold (4%, 20 nl) to SCN and allowed to rest for 10 days. The rats were then perfused with 4% paraformaldehyde and the brains were dissected for immunohistochemistry. Dopamine neurons projecting to SCN were identified with double immunochemistry of fluorogold (FG) and tyrosine hydroxylase (TH). To verify functional connection, dopamine metabolites at SCN were measured by microdialysis in freely moving rats following infusion of AMPA (0.3 mM/0.5 □1) to SuMN.

### Results:

Following infusion of retrograde tracer fluorogold at SCN, fluorogold immunoreactivity was identified in many brain regions that are known to receive direct innervations from SCN such as medial preoptic area (MPO), subparaventricular zone (SPVZ), dorsomedial hypothalamic nucleus (DMH), periventricular hypothalamic nucleus (Pe), paraventricular nucleus (PVN), zona inserta (ZI), arcuate nucleus (Arc), median eminence (ME), ventrocaudal posterior hypothalamus (PH), supramammillary nucleus (SuMN) and lateral parabranchial nucleus (LPB) and nucleus of solitary tract (NST).

Neurons dual-immunoreactive for fluorogold and tyrosine hydroxylase were observed almost exclusively in the SuMN and adjacent ventrocaudal posterior hypothalamus. A few scattered FG/TH dual-labeled neurons were found in Pe. Very few FG/TH dual labeled neurons were found in the hypothalamic nuclei that are known to be involved in energy metabolism such as PVN, DMH and Arc. Similar results were obtained in double labeling studies in Syrian hamster. Additionally in hamsters, significant numbers of FG/TH dual labeled neurons were detected in LPB.

The double-labeling results indicate that dopamine neurons in SuMN and the adjacent ventrocaudal posterior hypothalamus are the main source of dopamine input at SCN.

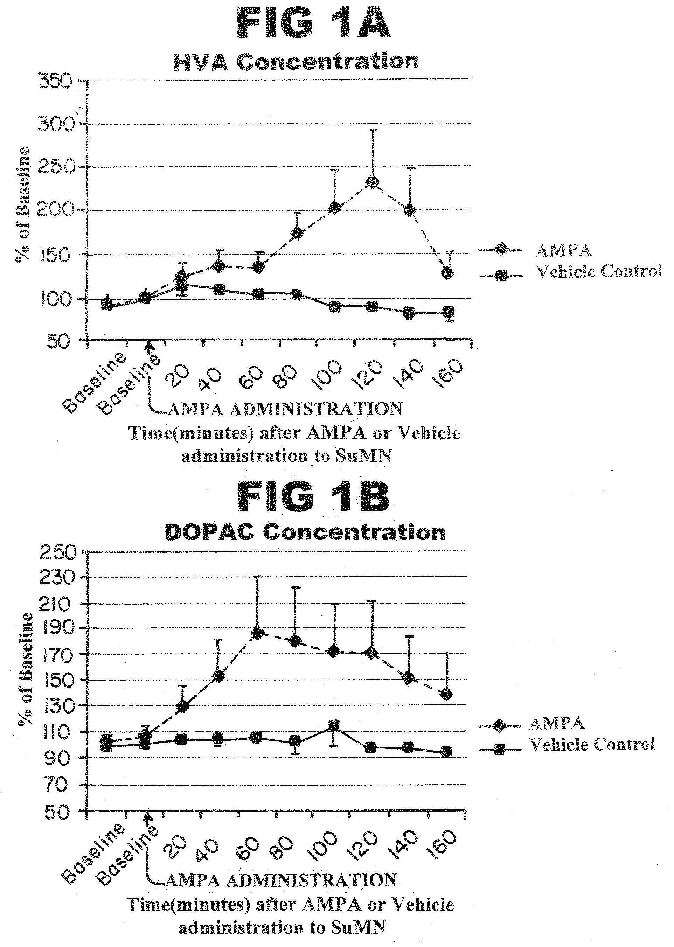

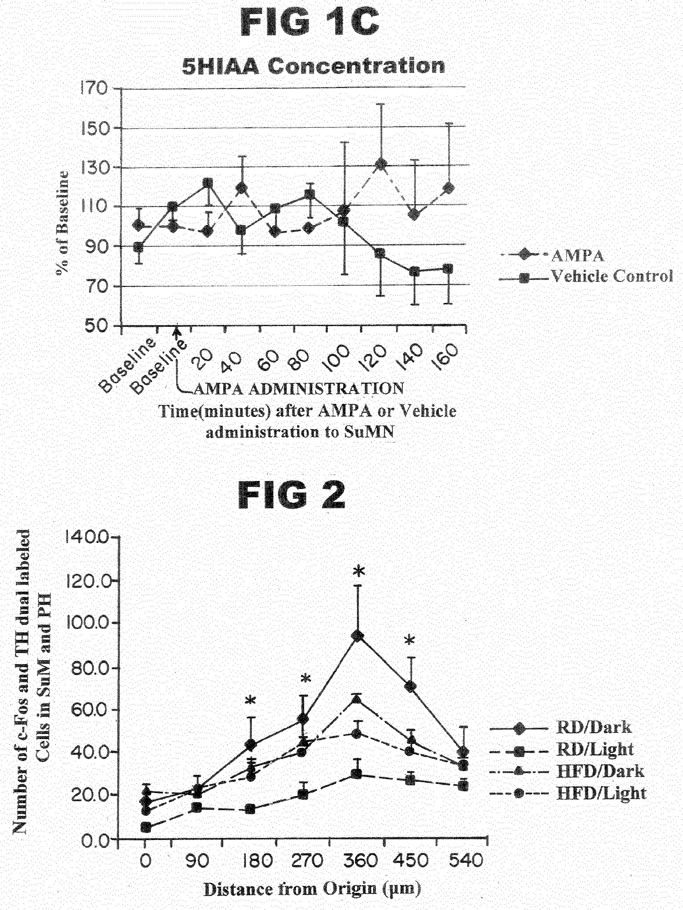

Results shown in FIG 1 demonstrate that stimulation of dopamine neurons in SuMN by intra-SuMN AMPA infusion produced significant elevations of dopamine metabolites HVA (FIG 1A; p < .05) and DOPAC (FIG 1B; p < 0.05) at SCN area compared to vehicle. AMPA infusion did produce a significant elevation of the serotonin metabolite 5-HIAA at the SCN (FIG 1C), compared to vehicle. These results verified a functional connection between dopamine release by SuMN neurons and a rise in dopamine in the SCN.

In summary, dopamine neurons in SuMN project to and release dopamine at SCN.

### Example 2: High fat diet feeding reduces the rhythmic activity of dopamine neurons in SuMN

### Protocol

Female Sprague-Dawley were fed high fat diet (HFD) or regular diet ad libitum and maintained on a 14 hour daily photoperiod. After 4 months of feeding, 10 rats on regular diet and 10 rats that became obese due to high fat diet feeding were selected for microdialysis study. At the end of microdialysis experiment, rats were sacrificed by trans-cardiac perfusion of 4% paraformaldehyde at 4 hours after light onset (4 "HALO") 16 HALO, respectively. The brains were dissected for double immunohistochemistry of c-Fos and tyrosine hydroxylase. The number of neurons double immunopositive for c-Fos and tyrosine hydroxylase in SuMN were counted as an indicator of activity of dopamine neurons in SuMN.

### Results

The results shown in FIG 2 demonstrate a circadian rhythm for dopamine expressing neurons in the SuMN, and that this circadian rhythm was disrupted in obese rats fed a fat diet. Non-obese rats fed a regular diet exhibited an increase in the number of dual labeled c-Fos/TH-expressing dopamine neurons in SuMN in the dark, i.e., 16 HALO compared to in the light, 4 HALO. In high fat diet-induced obese rats, the night peak of c-Fos expressing dopamine neurons in SuMN was reduced and the level of c-Fos expressing dopamine neurons in SuMN was elevated in the light. *See* FIG 2.

### EXAMPLE 3: Chronic activation of dopamine neurons in SuMN with AMPA reduces high fat diet-induced obesity/metabolic abnormalities

### Protocols

In separate protocols, female Sprague-Dawley high fat diet -resistant rats (HF-R) (16 animals) were fed high fat diets for 4 months or female Sprague-Dawley high fat diet-sensitive rats (HF-S) (16 animals) were fed high fat diets for 3 months. Following initial feeding protocols, AMPA (0.03 mM/0.5 □1) or vehicle was microinjected into SuMN daily for 13 (HF-R animals) or 14 d (HF-S) at the onset of locomotor activity. At the end of AMPA treatment, HF-R animals were sacrificed by decapitation. Trunk blood was collected for glucose and insulin analysis retroperitoneal fat mass were weighed. At the end of AMPA treatment, HF-S animals were subjected to glucose tolerance tests (GTT) and two days after GTT, were sacrificed. Trunk blood was collected for glucose and insulin analysis and retroperitoneal fat mass were weighed. Body weight and food consumption were measured daily throughout both protocols.

### Results

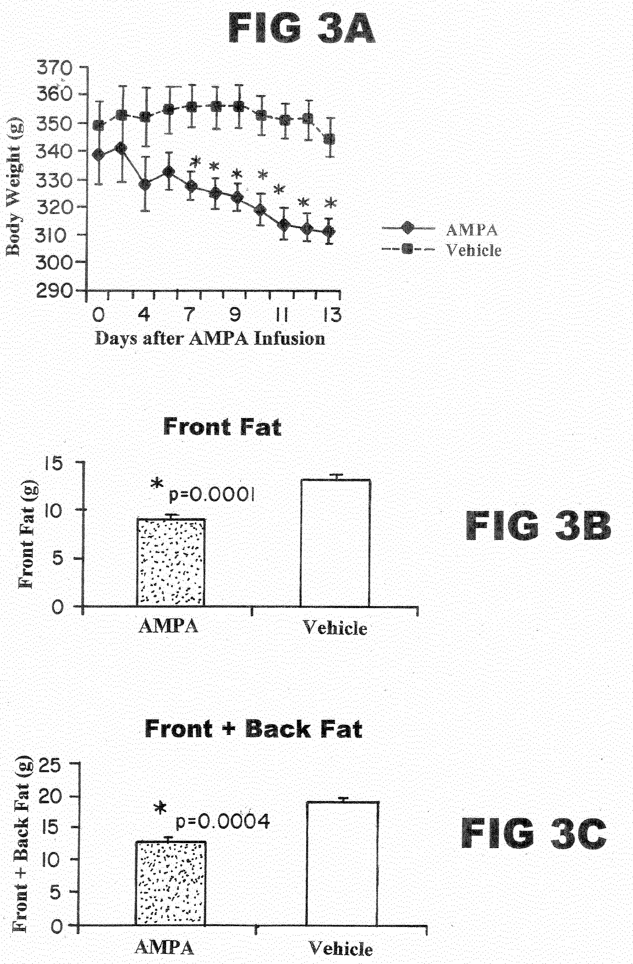

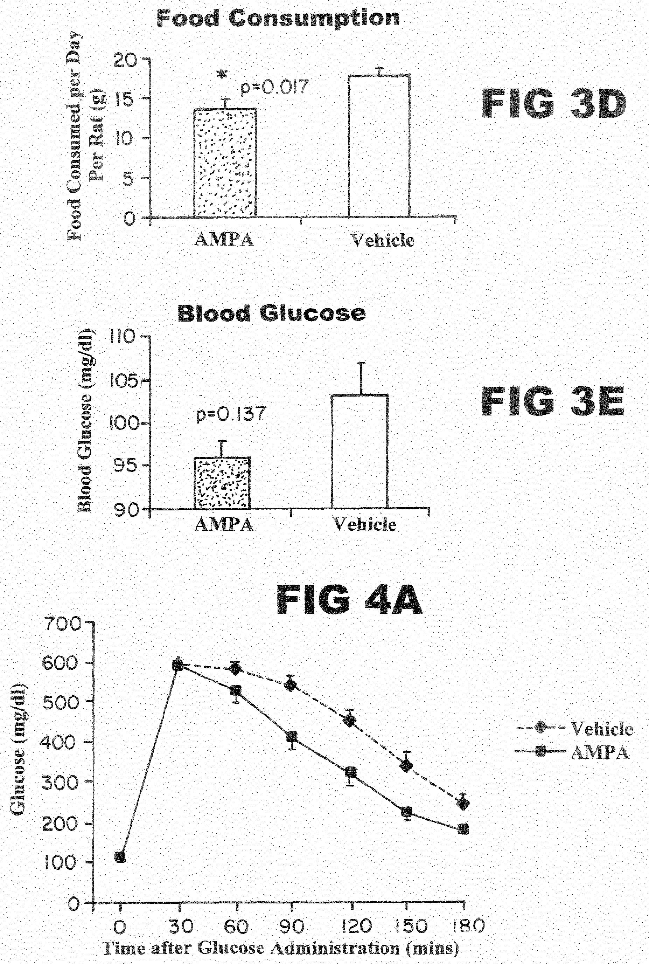

FIG 3 shows the results of chronic AMPA treatment on HF-R rats. Chronic AMPA treatment (N=5 AMPA treated; N=6 vehicle) significantly reduced body weight (p< 0.05), retroperitoneal fat 33% (13.4 g □ 0.48 versus 9.02g □ 0.48; p<0.01) and total abdominal fat 32% (19.1g □ 0.8 versus 12.9 g □ 0.6 ; p<0.01) in the HF-R rats. A small but significant reduction in daily food consumption was also detected (17.7 g □ 2.1 versus 13.7 g □ 1.0; p <0.05). Although statistically not significant, a reduction in fasting blood glucose was also detected (96.1□ 1.97 versus 103.3 □ 3.68, p=0.14).

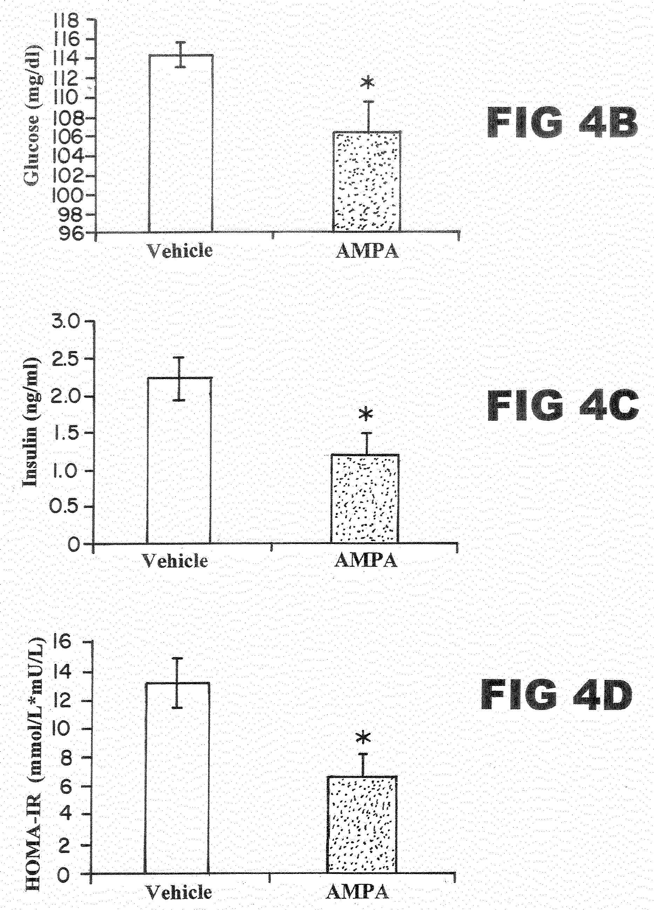

FIG 4 shows the results of chronic AMPA treatment on HF-S rats. Intra-SuMN AMPA treatment significantly reduced plasma glucose concentration during the GTT, compared to control animals treated with vehicle. The basal plasma glucose and insulin levels were both decreased by AMPA treatment (from 114 to 106 mg/dl and from 2.2 to 1.15 ng/ml, respectively, P<0.05). Insulin resistant index (HOMA-IR) analysis showed intra-SuMN AMPA reduced insulin resistance (from 13.0 ± 1.79 to 6.3 ± 1.70, p = 0.018). The total area under the glucose GTT curve also decreased. The plasma insulin level during the GTT is also expected to be significantly decreased by AMPA treatment.

Together, these results demonstrated that daily activation of SuMN with AMPA reduced obesity and glucose intolerance/insulin resistance induced by high fat feeding.

### Example 4: Chronic inhibition of dopamine neurons in SuMN with CNQX (AMPA receptor antagonist) and muscimol (GABA_{A} receptor agonist) induce obesity/metabolic abnormalities

### Protocol

The effect of blocking the rhythmic elevation of dopamine at SCN on energy balance was examined. The rhythmic activity peak of dopamine neurons in SuMN was chronically inhibited by daily infusion of CNQX (AMPA receptor antagonist) and muscimol (GABA_{A} receptor agonist) chronically for 10 days.

Female Sprague-Dawley rats were fed a high diet ad libitum. High fat diet resistant (HF-R) animals (24) were chronically infused with a solution containing of a mixture of an AMPA receptor antagonist, CNQX (0.15 mM), and a GABA_{A} receptor agonist, muscimol (0.1 mM), or vehicle at a rate of 0.5 □1/h, chronically for 10 days. Body weight and food consumption were measured daily. At the end of treatment, rats were sacrificed by decapitation. Trunk blood was collected for glucose and insulin analysis. Retroperitoneal fat was weighed.

### Results

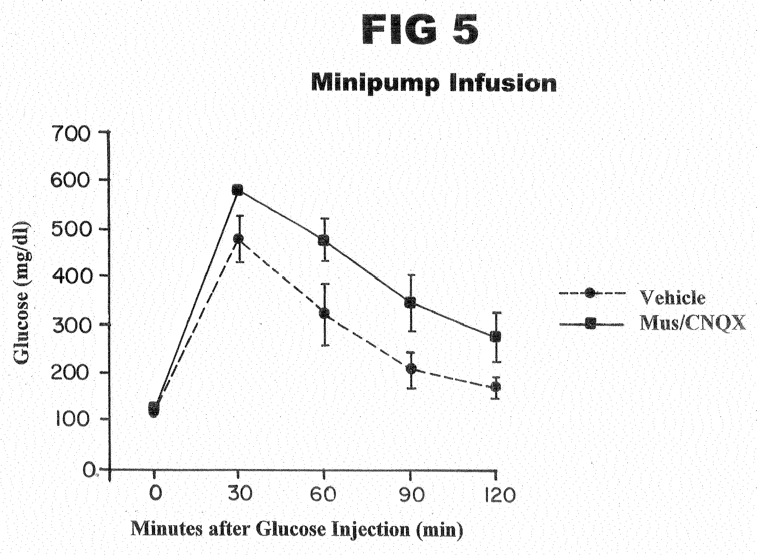

Results of a glucose injection are shown in FIG 5. Inhibition of dopamine neurons at SuMN by CNQX plus muscimol in HF-R animals lead to glucose intolerance.

### Example 5: Inhibition of dopamine neurons in SuMN with AMPA receptor antagonist (CNQX) and GABA_{A} receptor agonist (muscimol) reduces light pulse-induced c-Fos expression at SCN

### Protocol

To determine whether inhibiting dopamine input to SCN area could alter the photosensitivity of SCN, the dopamine neurons in SuMN was inhibited by intra-SuMN infusion of CNQX and muscimol.

Female Sprague-Dawley rats (20) were fed regular diet ad libitum. One day before the experiment, rats were divided into 4 groups (N=5 per group) and transferred to a room with dim red light. Rats received infusion of 0.5 □l of the mixture of CNQX( 0.1 mM) and muscimol (0.7 mM) or vehicle at one hour before the onset of locomotor activity (13 HALO) again at the onset of locomotor activity (14 HALO), under dim red light. At 15 HALO, half of the rats (5 rats treated with CNQX/muscimol and 5 vehicle rats) were exposed to a 30 min light pulse (∼200 lux) and the other half were kept under dim red light. One hour after the beginning of light pulse, rats were sacrificed by trans-cardiac perfusion of 4% paraformaldehyde. The brains were dissected for immunohistochemical staining of c-Fos. The expression level of c-Fos at SCN was quantified.

### Results

Light pulse induced a robust c-Fos expression at SCN. The level of c-Fos expression was significantly reduced within the core of the SCN in CNQX/muscimol treated rats.

These results demonstrated that inhibition of the activity of dopamine neurons in SuMN altered the photosensitivity of SCN to light pulse.

### Example 6.- Enhancing Circadian Dopamine Transmission at the SuMN Improves High Fat Diet (HFD)-Induced Glucose Intolerance/Insulin Resistance

Treatment of insulin resistant high fat fed rats with an NMDA agonist directed to the SuMN improved insulin resistance and glucose intolerance. Female Sprague Dawley rats were fed a high fat diet for one month and those animals most sensitive to the diet (those gaining the most weight in the group) were then treated with either an NMDA agonist or vehicle for a three minute period at the offset of light (14 hour daily photoperiod) via a cannula directed to the SuMN daily for 2 weeks while maintained on the high fat diet. At the end of the treatment period, animals were subjected to a glucose tolerance test (GTT) (3g/kg of glucose) and plasma samples were taken before and at 30, 60, 90, and 120 minutes after glucose treatment. Plasma samples were analyzed for glucose and insulin levels.

### Results

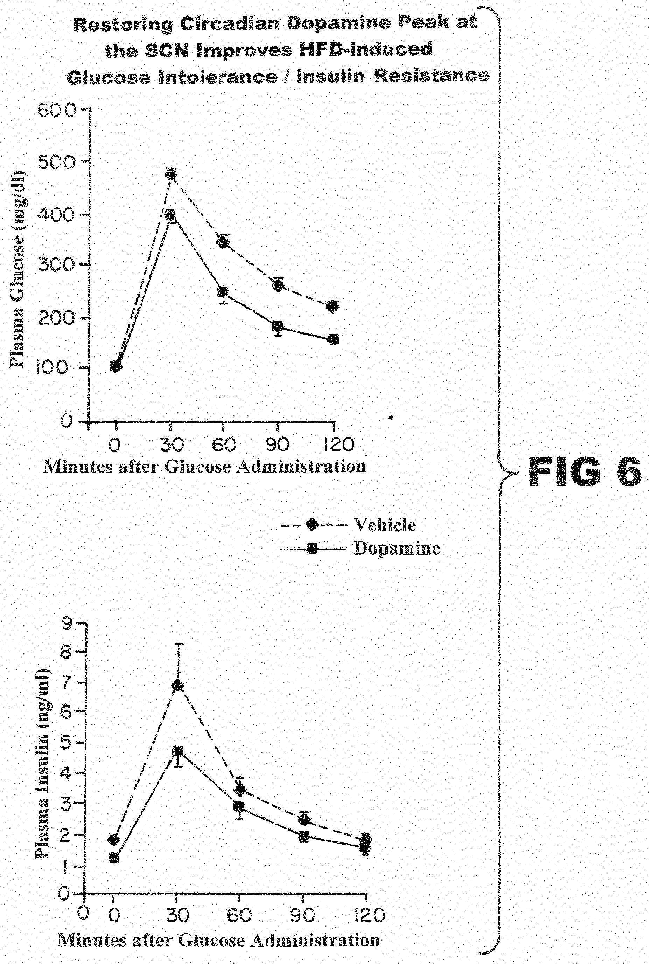

NMDA treatment significantly reduced plasma glucose and insulin levels during the GTT and significantly improved insulin resistance relative to control vehicle treated high fat fed animals. See Figure 6 for details of results.

### Example 7. Restoring Circadian Dopamine Peak at the SCN Improves HFD-Induced Glucose Intolerance/Insulin Resistance

Infusion of dopamine directly into the SCN of insulin resistant rats on a high fat diet improved insulin resistance and glucose intolerance. Male SHR rats at approximately 12 weeks of age were fed a high fat diet for one month and were then treated with either dopamine or vehicle for a three minute period at the offset of light (14 hour daily photoperiod) via a cannula directed to the SCN daily for 2 weeks while maintained on the high fat diet. At the end of the treatment period, animals were subjected to a glucose tolerance test (GTT) (3g/kg of glucose) and plasma samples were taken before and at 30, 60, 90, and 120 minutes after glucose treatment. Plasma samples were analyzed for glucose and insulin levels.

### Results

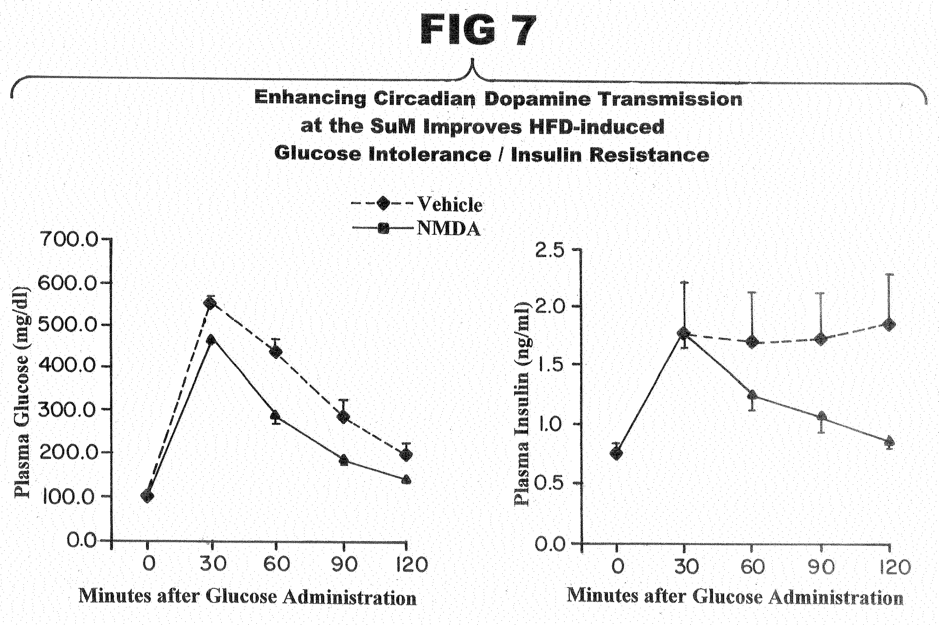

NMDA treatment significantly reduced plasma glucose and insulin levels during the GTT and significantly improved insulin resistance relative to control vehicle treated high fat fed animals. See Figure 7 for details of results.

Thus it can be seen that treatment with AMPA or NMDA to activate glutamate AMPA receptors or NMDA receptors at the SuMN at the onset of locomotor activity reduces high fat diet-induced obesity, glucose intolerance/insulin resistance, while blocking this glutamate receptor activity induces the glucose intolerant condition. Likewise addition of dopamine directly to the SCN improves insulin resistance and glucose intolerance.

A number of embodiments of the invention have been described. Nevertheless, it will be understood that various modifications may be made without departing from the spirit and scope of the invention. Accordingly, other embodiments are within the scope of the following items.

In specific embodiments the invention also relates to the following items:

### ITEMS

1. A method of treatment comprising administering an agent that increases neuronal activity in the supramammillary nucleus (SuMN) to a subject suffering from a metabolic disorder or a key element thereof, to treat said metabolic disorder or the key element.
2. The method of item 1 wherein the agent comprises at least one of an AMPA receptor agonist, an NMDA receptor agonist and/or a GABAₐ antagonist
3. The method of item 1 wherein said agent increases dopamine release from a dopaminergic neuron in the supramammillary nucleus (SuMN).
4. The method of item 1 wherein said dopaminergic neuron comprises projections that project to the area of the suprachiasmatic nucleus (SCN) of said subject.
5. The method of item 4 wherein said projections release dopamine into the SCN of said subject.
6. The method of item 2 wherein said metabolic disorder or key element thereof is type 2 diabetes, pre-diabetes, cardiovascular disease, or obesity.
7. The method of item 6 wherein said agent is an AMPA receptor agonist, an NMDA receptor agonist, or a GABA_{A} antagonist.
8. The method of item 1 wherein said agent is administered at a predetermined time such that said agent increases dopamine release from said dopaminergic neuron in the SuMN at about the time of wakening of said subject.
9. The method of item 8 wherein said agent is administered to said patient within about 2 hours of wakening.
10. A method of treatment comprising administering to a patient suffering from a metabolic disorder or a key element thereof an AMPA receptor agonist and/or an NMDA receptor agonist, to treat said a metabolic disorder or a key element thereof.
11. The method of item 10 wherein said metabolic or key element thereof is type 2 diabetes, pre-diabetes, cardiovascular disease, or obesity.
12. The method of item 11 wherein said AMPA receptor agonist and/or NMDA receptor agonist is administered a predetermined time such that said agonist is active at AMP receptors in the SuMN of said patient at about the time of awakening of said patient.
13. The method of item 12 wherein said AMPA receptor agonist and/or NMDA receptor agonist is administered to said patient within about two hours of awakening.
14. A method of treatment comprising administering a GABA_{A} antagonist to a patient suffering from a metabolic disorder or a key element thereof, to treat said a metabolic disorder or a key element thereof.
15. The method of item 14 wherein said metabolic or key element thereof is type 2 diabetes, pre-diabetes, cardiovascular disease, or obesity.
16. The method of item 15 wherein said GABA_{A} antagonist is administered a predetermined time such that said antagonist is active at GABA_{A} receptors in the SuMN of said subject at about the time of awakening of said patient.
17. The method of item 16 wherein said AMPA receptor agonist and/or NMDA receptor agonis is administered to said patient within about two hours of awakening.
18. A method of treatment comprising contacting a neuron in the SuMN in a patient in need of treatment of a metabolic disorder or key element thereof with an agent that increases AMPA receptor and/or NMDA receptor activity of said neuron, to increase baseline activity of said AMPA receptor and/or NMDA receptor activity, to treat said a metabolic disorder or a key element thereof.
19. A method of treatment comprising contacting a neuron in the SuMN in a patient in need of treatment of a metabolic disorder or key element thereof with an agent that decreases gabanergice receptor activity of said neuron, to decrease baseline activity of said gabanergic receptor activity, to treat said a metabolic disorder or a key element thereof.
20. A method of treating a metabolic disorder or a key element thereof comprising increasing the ratio of AMPA receptor activity and/or NMDA receptor activity to gabanergic receptor activity in the SuMN or an individual suffering from said a metabolic disorder or a key element thereof, to treat said a metabolic disorder or a key element thereof.
21. A method of treating a metabolic disorder or a key element thereof in a subject suffering from therefrom, comprising administering to said subject a first amount of an AMPA receptor agonist and/or NMDA receptor agonist and a second amount of a GABA_{A} antagonist wherein said first and second amounts together comprise a therapeutically effective amount of an active combination of agents to treat said a metabolic disorder or a key element thereof.
22. A method of normalizing a daily rhythm of dopamine release in the SuMN comprising administering an AMPA receptor agonist and/or NMDA receptor agonist and/or a GABA_{A} antagonist to a subject suffering from an aberration of said daily rhythm.
23. The method of item 22 wherein said AMPA receptor agonist and/or NMDA receptor agonist and/or GABA_{A} antagonist is administered at a predetermined time such that said agonist or GABA_{A} antagonist is active at receptors in the SuMN of said subject at about the time of the onset of locomotory activity of said subject.
24. The method of item 22 wherein said AMPA receptor agonist and/or NMDA receptor agonist and/or GABA_{A} antagonist is administered at a predetermined time such that said agonist or GABA_{A} antagonist is active at receptors in the SuMN of said subject at about the time of the awakening of said subject.
25. The method of item 22 wherein said AMPA receptor agonist and/or NMDA receptor agonist and/or GABA antagonist is administered to said patient within about two hours of awakening.
26. A method of stimulating dopamine release from dopaminergic neurons in the SuMN of a subject comprising administering an AMPA receptor agonist and/or NMDA receptor agonist and/or a GABA_{A} antagonist to said subject.
27. A pharmaceutical composition comprising an AMPA receptor agonist, an NMDA receptor agonist, and/or a GABA_{A} antagonist and a pharmaceutically acceptable excipient.
28. A method of treating a metabolic disorder or a key element thereof comprising adjusting an aberrant daily rhythm of dopamine release in the SuMN of a subject in need of treatment of said metabolic disorder or a key element thereof to approach a normal daily rhythm of dopamine release a dopaminergic neuron in the SuMN of normal individuals of the same species and gender as said subject, who do not suffer from said metabolicdisorder or a key element thereof.
29. A method of treatment comprising administering an AMPA receptor agonist and/or NMDA receptor agonist and/or a gabanergic receptor antagonist to a subject suffering from a metabolic disorder or a key element thereof, to increase CNS dopamine activity at about the time of day of that is the time of day that dopamine activity peaks in the CNS of healthy individuals of the same species and gender, to treat said a metabolic disorder or key element thereof.
30. The method of item 29 wherein said metabolic disorder or key element thereof is type 2 diabetes, pre-diabetes or obesity.
31. The method of item 30 wherein said AMPA receptor agonist and/or NMDA receptor agonist and/or gabanergic receptor antagonist is administered a predetermined time such that said agonist or antagonist is active at receptors in the SuMN of said subject at about the time of the onset of locomotory activity of said subject.
32. The method of item 31 wherein said AMPA receptor agonist and/or NMDA receptor agonist and/or gabanergic receptor antagonist is administered at a predetermined time such that said agonist or antagonist is active at receptors in the SuMN of said subject at about the time of awakening of said subject.
33. The method of item 30 wherein said predetermined time is within about two hours of awakening.
34. A method of treatment comprising administering an NMDA receptor agonist to a patient suffering from a metabolic disorder or a key element thereof, to treat said metabolic disorder or a key element thereof.
35. The method of item 34, wherein said metabolic disorder or key element thereof is type 2 diabetes, pre-diabetes, cardiovascular disease, or obesity
36. A method of treatment comprising administering a blood brain barrier penetrating dopamine analog to a patient suffering from a metabolic disorder or a key element thereof, to treat said metabolic disorder or a key element thereof.
37. The method of item 36 wherein said metabolic disorder or key element thereof is type 2 diabetes, pre-diabetes, cardiovascular disease, or obesity
38. A method of treatment comprising administering a blood brain barrier penetrating dopamine analog to a patient suffering from a metabolic disorder or a key element thereof, to treat said metabolic disorder or a key element thereof.
39. Method of item 38 wherein the blood brain barrier penetrating dopamine analog is administered between 0400 to 1200 hours.
40. Method of item 39 wherein the blood brain barrier penetrating dopamine analog is administered within 2 hours of waking.
41. The method of item 38 wherein said metabolic disorder or key element thereof is type 2 diabetes, pre-diabetes, cardiovascular disease, or obesity.
42. The method of item 41 which comprises administering said blood brain barrier penetrating dopamine analog to said patient at about the time of awakening of the patient.

## Claims

1. A composition comprising a blood brain barrier penetrating dopamine analog for use in treatment of a patient suffering from type 2 diabetes, pre-diabetes, cardiovascular disease, or obesity.

2. A composition for use according to claim 1 wherein said analog is administered at a predetermined time such that said agent increases dopamine release from said dopaminergic neuron in the SuMN at about the time of wakening of said subject.

3. A composition for use according to claim 2 further including an AMPA receptor agonist selected from the group consisting of (RS)-α-Amino-3-hydroxy-5-methyi-4-is¬oxazolepropionic acid hydrobromide ("(RS)-AMPA hydrobromide"); (R)-α-Amino-3-hydroxy-5-methyl-4-iso-xazolepropionic acid ("(R)-AMPA"); (RS)-α-Amino-3-hydroxy-5-methyl-4-is¬oxazolepropionic acid ("(RS)-AMPA"); (S)-α-Amino-3-hydroxy-5-methyl-4-iso¬xazolepropionic acid ("(S)-AMPA"); RS)-2-Amino-3-(4-chloro-3-hydroxy-¬5-isoxazolyl)propionic acid ("CI-HIBO"); (S)-α-Amino-2,3,4,5,6,7-hexahydro-2,4-dioxo-1H-cyclopentapyrimidine-1-propanoic acid ("(S)-CPW 399"); (S)-(-)-α-Amino-5-fluoro-3,4-dihydro-2,4-dioxo-1(2H)pyridinepropanoic acid ("(S)-(-)-5-Fluorowillardiine"); NPEC-caged-(S)-AMPA ("(N)-1-(2-Nitrophenyl) ethylcarboxy-(S)-α-1-(2 nitrophenyl)ethylcarboxyamino-3-hydroxy-5-methyl-4-isoxazolepropionic acid"); and (L)-(+)-α-Amino-3,5-dioxo-1,2,4-oxadiazolidine-2-propanoic acid ("L-Quisqualic acid") (S)-2-amino-3-(3-hydroxy-5-phenylisoxazol-4-yl)propionic acid ((S)-APPA), 2-pyridyl analogue of APPA, (RS)-2-amino-3-[3-hydroxy-5-(2-pyridyl)isoxazol-4-yl]propionic acid (2-Py-AMPA); 5-HPCA; N,N'-(2,2'-(1,4-phenylene)bis(ethane-2,1-diyl))dipropane-2-sulfonamide; and AMPA Activator (CMPA).

4. A composition for use according to claim 1 further including a GABAA antagonist.

5. A composition for use according to claim 1 further including an NMDA receptor antagonist.

6. A composition for use according to claim 1 wherein said dopamine analog is administered at a predetermined time such that said analog increases dopamine release from said dopaminergic neuron in the SuMN at about the time of wakening of said subject.

7. A composition for use according to claim 6 wherein said composition is administered to said patient between 0400 to 1200 hours.

8. A composition for use according to claim 6 wherein said composition is administered to said patient within 2 hours of waking.

9. A composition for use according to claim 1 administered to the patient at about the time of awakening of the patient.
